# EUROPEAN PATENT APPLICATION

(11) **EP 0 581 750 A1**
(43) Date of publication of application: **02.02.1994**
(21) Application number: 93850150.9
(22) Date of filing: 19.07.1993
(51) Int. Cl.: A61C 1/08, A61B 17/36

(54) **Device for the emission of laser light**

(30) Priority: 20.07.1992 SE 9201754
(71) Applicant: Gustavsson, Morgan, S-413 01 Göteborg (SE)
(72) Inventor: Gustavsson, Morgan, S-413 01 Göteborg (SE)
(74) Representative: Mossmark, Anders

(57) **Abstract**

The present invention relates to a device for emission of laser light. The invention is characterized in that the device comprises one for the hand adapted longitudinal handle housing a laser light emitting crystal unit (1) between a front (4) and a rear (3,21) mirrors and supply devices (16,23) for energy to the laser crystal (1) and outlet devices (9,18) for emitted light or laser light.

## Description

### TECHNICAL FIELD:

The present invention relates to a device for the emission of light or laser light for treatment of material in the body of a human or an animal. It relates specifically to a hand held device intended to create desired light or laser light and irradiate the part of the body which is intended to be treated.

### PRIOR ART:

It is known earlier that irradiation from an erbium laser is useable for influencing material in the body, for example hard tissue as cartilage, bone, enamel and dentine and soft tissue as muscles, fat, vessels and nerves.

To bring about this irradiation a stationary or moveable device comprising an erbium crystal which is supplied with energy and which create laser irradiation of desired wave length is used. This device for creation of the laser light has a size which makes it unhandy for treatment of the patient and it is therefore arranged at a certain distance from him. The light must therefore be transported from this light emitting erbium crystal to the target for the irradiation. The transport of this light occurs by means of a light conductor consisting of a jointed mirror reflexing arm or by a hollow bendable, relatively stiff light conductor. It can also consist of an optical fibre or a fibre bunch.

### THE TECHNICAL PROBLEM:

A light conductor of the above described kind is unconvenient, relatively expensive and gives rise to substantial effect losses. This occurs specially if the light conductor is a jointed mirror reflex arm or is of the type which is hollow, bendable but relatively stiff. They do also have the disadvantage that they are shortlived depending on brittleness and hygroscopic properties and very expensive besides creating substantial effect losses. It is specially light conductors consisting of optical fibres that have these disadvantages.

Erbium laser equipments are today characterized in that they are very costly for hospitals etc. This limits today very much the availability of erbium laser treatment for different categories of treatment for example pain free dental drilling where erbium laser has shown excellent properties.

It has therefore long been a desire to create erbium laser light and non-laser light from erbium crystals more available to patients and doctors.

### THE SOLUTION:

One has accordingly wanted to make a laser device without the above said disadvantages and according to the invention one has therefore brought about a device for the emission of light or laser light which is characterized in that it comprises a for the hand adapted longitudinal handle housing a laser light emitting crystal unit between a front and a rear mirror and supply arrangements for energy to the laser crystal and output arrangements for emitted light or laser light.

It is according to the invention's advantageous that the laser crystal unit consists of an erbium crystal unit.

According to the invention the supply arrangements for energy comprise a device for supply of electrical energy having a high voltage, electric energy having a low voltage or light.

It is further according to the invention suitable that the laser crystal unit is supplied with light energy laterally from a lamp or that it is supplied at one end from a diode.

It is according to the invention also possible that the laser crystal unit can be supplied with light energy at one end coming from a laser, diode or lamp at some distance from the handheld device.

The output arrangement for emitted light can according to the invention comprise a light directing piece having mirrors and lenses or optical fibres or a hollow light conductor.

The erbium crystal can for example consist of Er:YAG (Erbium : Yttrium, Aluminium, Garnett), ErCr:YAG (Erbium, Krom : Yttrium, Aluminium, Garnett), Er:YSGG (Erbium Yttrium, Solmium, Garnett, Glas) or ErCr:YSGG (Erbium, Krom : Yttrium, Solmium, Garnett, Glas).

By the new location of a light emitting erbium crystal the transport need of its emitted light is diminished to such an extent that the above described problems with light conductors are not only solved but de facto eliminated.

By the present invention both erbium laser and non-laser light from an erbium crystal can be made more available for patients and doctors.

This is not only from a practical user's view preferable but also highly economical. The cost gain and hence the double increased availability lies partly in the saving of the relatively expensive erbium light conductor, partly in that the energy losses up to above 50% in this one is eliminated. The elimination of these energy losses means that smaller and hence cheaper components of such elements as voltage aggregates, light source, erbium crystal, cooling device, transport system etc can be used.

### FIGURE DESCRIPTION:

The invention will in the following be described more in detail in connection with the attached figures where
Fig. 1 shows a device according to the invention in practical use,
Fig. 2 in section shows an embodiment of the invention,
Fig. 3 shows in section another embodiment of the invention,
Fig. 4 shows a third embodiment of the invention,
Fig. 5 shows a fourth embodiment of the invention and where
Fig. 6 in section shows a fifth embodiment of the invention.

### PREFERRED EMBODIMENTS:

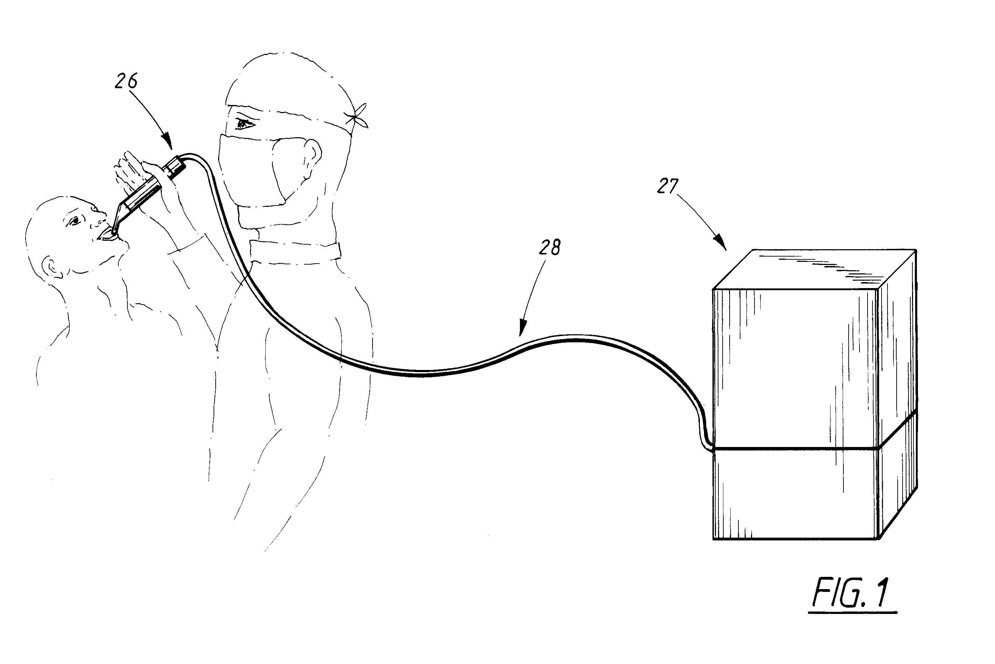

Fig. 1 shows a dentist who uses the device according to the present invention for treating the teeth of a patient. The erbium light which shall beam out against the teeth of the patient is produced in the handpart 26 i.e. closest possible to the target of the light. Any light conductor for the light emitted from the erbium crystal out to that part which the operator carries in his hand is therefore not needed. The electrical energy supply and in some cases photonic energy supply, energy controling equipment, tank with cooling medium, one or two pumps and filter for one or two cooling media etc is located in one or more separate boxes 27.

Energy and light sources is known technique and is marketing in a great number of variants by a plurality of companies. The same applies to reflectors, erbium crystals, pumps, filters etc. Variations in that kind of technique is of no real importance for the invention and is therefore not dealt with closer here.

The connection between the handpart 26 and the box 27 occurs via a multi conduction cable 28. This cable 28 contains conductors in both directions for cooling medium and conductors in both directions for electricity. In some cases it also contains a conductor for light having a lower wavelength than the light which is emitted from the erbium crystal and which is in relation to the erbium crystal light uncomplicated to transmit via a light conductor.

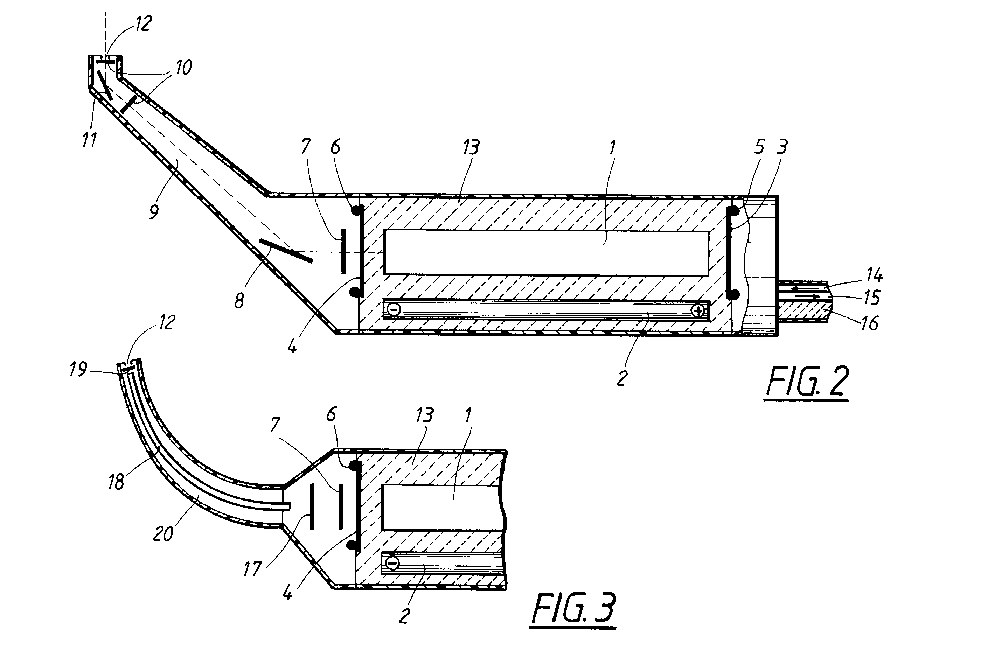

Fig. 2 and 3 show the same type of device. They have the same lateral, optical pumping principle, which means that they are supplied with light from the side, and differ only in the laser output part to the left. On the figures the erbium crystal is designated with 1 and the optical resonator consists of the space between the mirrors 3 and 4. The handpart is in its outer configuration a round and/or elliptic cylinder in section having a light output piece 9, 20, 18 or 20 plus 18 for light beams in its one end.

The angle of the light output piece which is shown on the figures is intended to make it easier to come closer to the target for the light. The light output piece can consist of a corridor for the light beam as shown on fig. 2 with mirrors 8 and 11 and converging lenses 10 and 12.

The angle of the light output piece is not necessary and a straight beam direction from the erbium crystal is both possible and simpler from a construction point of view.

The light output piece can further consist of a lens coupling system that conducts the light into a hollow light conductor or preferably an optical fibre as appears from fig. 3. The hollow light conductor 18 can be stiff or flexible.

An optical fibre 18 of a few-times use is possible due to its very short length. This length is smaller than 300 mm and preferably smaller than 50 mm. That makes it cheap and therefore easy to replace without large costs.

The optical fibre 18 can further have a stiff conducting sleeve 20 or be without conducting sleeve and then be relatively flexible so that one with that can reach narrow and/or angled spaces, for example a tooth root canal. The light conductor 9 or 18 can in the outlet end have a mounted converging lense 19.

The light conductor 9 or 18 has in the outlet end an opening. From this opening the light irradiates either diverging or parallel from the end of the beam conductor. It is however preferable that the outgoing beam has a converging character having a focus of 0,0 mm to +30,0 mm from the end of the light conductor 9 or 18.

At the outlet end for the light conductors 9 or 18 also a water nozzle can be mounted which can give a thin film of cooling water against the target for the light, preferably in the outgoing focus of the light. This is however not shown on the figures. If a flashlamp pumping from the side is used such as shown in figures 2 and 3 the handpart has an inner reflector 13 which is directed inwardly against the laser crystal 1 and the lamp 2. The reflector 13 can be of an elliptical type. It can also be of a close coupling type, i.e. be of such a small volume as possible so close as possible between the erbium crystal and the lamp.

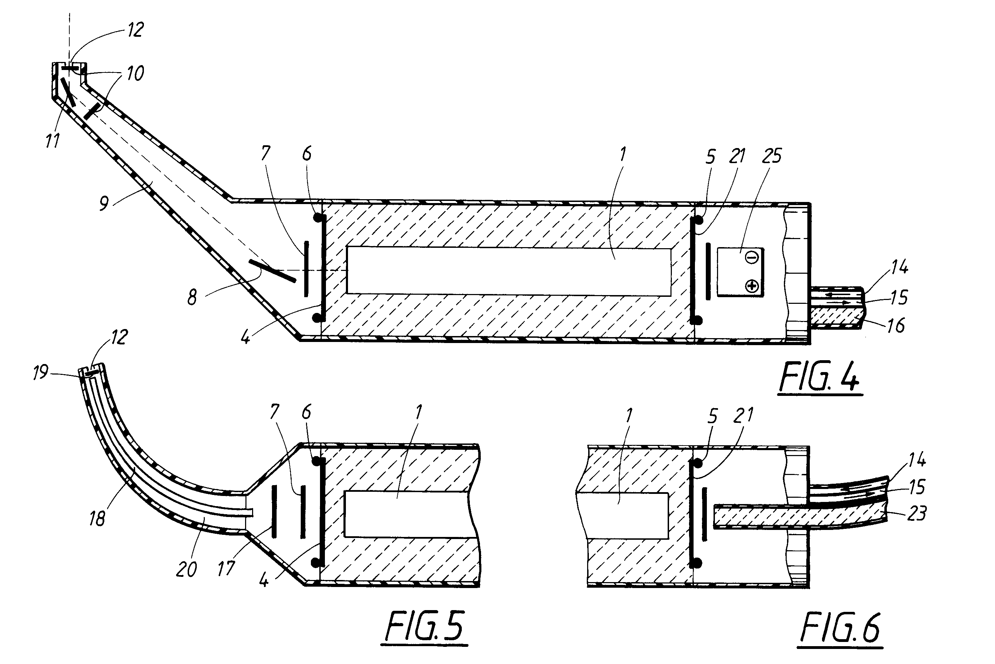

In the handpart an optical pump source consisting of one or more flashlamps 2 or consisting of one or more diodes 25 as shown on fig. 4 or one end of a light conductor 23 which is shown on fig. 5 which runs from a light source outside the handpart besides the laser crystal with resonator is included.

In the handpart is further located a cooling system for example an active or passive air or water cooling, an output part for the light beam with converging or parallelising lens system 7. There is also an outlet part for the water cooling for the target of the light beam which is not shown on the figures.

The same water can be used to cool the handpart and the target for the light beam.

The pumping light comes to the erbium crystal in preferably one of three ways which we have touched above:
1) Via one or more flashlamps 2 located along the erbium crystal 1 and a reflector 13 around these parts.
2) From one or more diodes 25 located outside the optical resonator of the erbium crystal and preferably behind this. The diodes can be of a lasering type or a not lasering type.
3) From a light source, preferably a diode or a flashlamp or some other laser emitting light of lower wave length than the lasering erbium crystal. This light source is located at a certain distance from the handpart, preferably in the same box 27 as the voltage aggregate. From there the pumping light is conducted via a light conductor 23, preferably an optical fibre or a fibre bunch or a hollow light conductor to preferably the rear end of the optical resonator of the erbium crystal 1.

To 1) and 2) the energy to the handpart will accordingly come in the shape of electricity via an electrical cable from a voltage aggregate located in the box 27 at some distance to the handpart. The current will return via the same cable.

To 3) the energy will accordingly come in the shape of light via a light conductor 23, preferably an optical fibre or a fibre bunch or a hollow light conductor from a light source preferably a lasering or a not lasering diode or a flashlamp or some other laser located at some distance from the handpart and preferably in the same box 27 as the voltage aggregate. Along the energy conductor for electricity or light out to the handpart one or in some case more conductors for cooling water and in some cases for air is running.

The optical resonator consists of a front mirror 4 and a rear mirror 3 or 21 (figs. 4 and 6). In some cases it can also consist of a reflecting sleeve around the erbium crystal.

Generally the function can be described in three partly divided principles:
Principle 1; The classical laser:
The laser crystal's dimensioning, the light pumping, the resonator with the partial permeability of the front mirror is adapted so that population inversion and stimulated emission is obtained. By population inverison is meant that more than 50% of the laser light emitting molecules in the crystals are in excited condition. The threshold of the lasering energy is exceeded by the energy amount per time unit coming from the voltage aggregate. Hereby a relatively parallel outgoing beam bunch usually 0,01-0,0001 steradianes depending on the mirror diameter and the length of the optical resonator is obtained.

Principle 2; The wave conductor laser:
This principle is as principle 1) above with population inversion and stimulated emission but with certain additions.

In these cases even the longitudinal limitation of the erbium crystal or its vicinity is reflecting.

Thus, besides the front and rear mirrors a reflecting sleeve or the difference in optical refraction index between the erbium crystal and the surrounding medium preferably air or water is used as internally reflecting medium for the erbium crystal.

In those cases the difference in the refraction index is used the internal reflector will not be 100% and a partial wave conductor laser is obtained. This gives a lesser beam cone from the front mirror than the one which is obtained by using the inwardly mirroring sleeve. It is usually smaller than or equal to the half of such a cone.

The inwardly mirroring sleeve is however not 100% either but its starting cone can be close to 180°. In these cases a larger spreading of the outgoing beam bunch usually in the order of size are greater than 60°, which is greater than 1 steradian.

This can, however, to a certain extent be used via lens focusing and/or some other condensing of the light before it has reached the target for the air. Due to less light energy being fed laterally from the erbium crystal the efficiency is increased and the need of cooling is decreased.

Principle 3; Not linear light conductor:
Also a spontaneous emission can be used if one does not get population inversion.

In such a case the laser crystal will function as a non linear wave conductor having either a reflecting sleeve or the difference in optical refraction index between the erbium crystal and surrounding medium preferably air or water as internal mirroring medium for the erbium crystal.

In those cases where the difference in the refraction index is used the inner internal reflector will not be 100% and a partial wave conductor laser is obtained.

This gives a lesser outgoing beam cone than the one which is obtained by using an inwardly mirroring sleeve which usually is less than or equal to the half of such a cone.

In these cases a larger spreading of the outgoing beam is obtained usually in the order of size greater than 60°, that is larger than 1 steradian.

However, an inwardly mirroring sleeve is neither completely 100% and its starting cone can be close to 180°.

The light in a such relatively wide cone can however to an appreciable extent be used via lens focusing and/or some other condensing of the light on the way to the target for the light. Due to less light energy being fed laterally from the erbium crystal the efficiency will be increased and the need for cooling decreased.

The outgoing light is continous and in some cases pulsating.

The pulse length of the outgoing light is regulated either by an electronic or mechanical shutter according to known technique or with the effect and the pulse length from the light source which in turn is regulated by the voltage aggregate with regulating electronics according to known techniques.

The length of the pulse can in dependence of the application area vary from one nano-second to one second whereupon the radiation is regarded as continous. For example, for tooth drilling preferably a pulse length which is under 500 microseconds, preferably 1-50 microseconds is used. The pulse can further be made up of a number of shorter pulses overlapping or not overlapping each other.

The rear mirror 21 which is shown on figs. 4 and 6 is in those cases where optical feeding from behind is used permeable for the light from the feeding light source but not permeable for the light from the crystal.

The invention is not limited to the embodiment examples shown but can be varied in different ways within the scope of the claims.

## Claims

1. Device for the emission of light or laser light characterized in that it comprises one for the hand adapted longitudinal handle housing a laser light emitting crystal unit (1) with a front (4) and a rear (3, 21) mirror and supply devices (16, 23) for energy to the laser crystal (1) and outlet devices (9, 18) for emitted light or laser light.

2. Device according to claim 1, characterized in that the laser crystal unit (1) is an erbium crystal unit.

3. Device according to claim 1 or 2, characterized in that the supply devices (16, 23) for energy comprises a device for supply of electrical energy having a high voltage, electrical energy having a low voltage or light.

4. Device according to any of the claims 1-3, characterized in that the laser crystal unit (1) is supplied with light energy laterally from a lamp (2).

5. Device according to any of the claims 1-3, characterized in that the laser crystal unit (1) is supplied with light energy at one end from a diode (22).

6. Device according to any of the claims 1-3, characterized in that the laser crystal unit (1) is supplied with light energy at one end from a laser diode or lamp at some distance from the hand part (26).

7. Device according to any of the claims 1-6, characterized in that the outlet device for emitted light comprises a light direction piece (9) having mirrors (8, 11) and lenses (7, 10) or optical fibres (18) or a hollow light conductor.
